# FASCICULE DE BREVET EUROPEEN

(11) **EP 4 153 763 B1**
(45) Date de publication et mention de la délivrance du brevet: **19.06.2024**
(21) Numéro de dépôt: 21727142.8
(22) Date de dépôt: 20.05.2021
(51) Int. Cl.: C12P 5/02, C12M 1/107, C12M 1/34, C12M 1/04, C12M 1/12

(54) **PROCÉDÉ DE MÉTHANATION DE L'HYDROGÈNE H2 ET DU DIOXYDE DE CARBONE CO2 OU DE L'HYDROGÈNE H2 ET DU MONOXYDE DE CARBONE CO EN VUE DE LA PRODUCTION DE MÉTHANE CH4**
VERFAHREN ZUR METHANISIERUNG VON WASSERSTOFF H2 UND KOHLENDIOXID CO2 ODER WASSERSTOFF H2 UND KOHLENMONOXID CO ZUR HERSTELLUNG VON METHAN CH4
METHOD FOR METHANATION OF HYDROGEN H2 AND CARBON DIOXIDE CO2 OR HYDROGEN H2 AND CARBON MONOXIDE CO FOR THE PRODUCTION OF METHANE CH4

(30) Priorité: 20.05.2020 FR 2005213
(43) Date de publication de la demande: 29.03.2023
(73) Titulaire: TMA-Process, 51100 Reims (FR); Institut des Sciences et Industries du Vivant Agroparistech, 75231 Paris (FR); Agro Industrie Recherches et Developpements A.R.D., 51110 Pomacle (FR); CentraleSupélec, 91192 - Gif sur Yvette Cedex (FR); Cristal Union, 10700 Veillette-sur-Aube (FR); GRTgaz, 92270 Bois-Colombes (FR)
(72) Inventeur: THEOLEYRE, Marc-André, 75020 Paris (FR); DESCHAMPS, Laure, 51100 Reims (FR); LEMAIRE, Julien, 08300 Saint-Loup-en-Champagne (FR); FILALI, Rayen, 51100 Reims (FR); IMATOUKENE, Nabila, 51450 Betheny (FR); MERLET, David, 51100 Reims (FR); PLANTEGENET, Audrey, 51100 Reims (FR); BELLOY, Christian, 08130 Saint Loup Terrier (FR); CLEMENT, Tiphaine, 31400 Toulouse (FR)
(74) Mandataire: Cabinet Bleger-Rhein-Poupon
(86) Numéro de dépôt international: PCT/EP2021/063433
(87) Numéro de publication internationale: WO 2021/234073

(56) Documents cités:
- EP-A1- 2 771 472
- EP-A1- 3 418 371
- WO-A1-2019/034820
- DE-A1-102018 213 950
- FR-A1- 2 601 690
- US-A1- 2019 309 256

## Description

La présente invention concerne un procédé de méthanation de l'hydrogène H₂ et du dioxyde de carbone CO₂ ou de l'hydrogène H₂ et du monoxyde de carbone CO, dans l'optique d'une production de méthane CH₄.

Le développement d'un tel procédé de production de méthane CH₄ s'inscrit dans un programme global dont le but est de développer des voies de stockage et de valorisation de diverses sources d'énergies renouvelables, notamment.

Par exemple, en ce qui concerne la production d'électricité renouvelable, à partir des énergies éolienne et solaire, sa dépendance aux conditions climatiques conduit à l'installation d'une surcapacité structurelle, et il est nécessaire de prévoir des moyens de stockage de l'électricité produite éventuellement en surplus.

On cite souvent, parmi de tels moyens de stockage de l'électricité, la production électrochimique d'hydrogène H₂.

Cet hydrogène H₂ peut être combiné avec du dioxyde de carbone CO₂, par exemple, par une réaction de méthanation, pour produire du méthane CH₄, qui serait injecté dans le réseau de distribution de gaz naturel. Il est ainsi envisageable de profiter de l'immense capacité de stockage constituée par le réseau de transport et de distribution de gaz.

Il existe notamment une voie biologique de méthanation aboutissant à une production de CH₄ par combinaison d'H₂ et CO₂, avec un dégagement de chaleur (réaction exothermique). La réaction de méthanation est alors plus particulièrement effectuée par une flore dite méthanogène hydrogénotrophe, notamment par des micro-organismes appartenant au domaine des Archae par exemple, et présente dans les fermenteurs de méthanisation.

De même, les voies métaboliques de fermentation de l'hydrogène H₂ et du monoxyde de carbone CO ont été identifiées chez des populations de microorganismes anaérobies (Munasinghe et Khanal, 2010).

En parallèle, se développent des voies de valorisation des biomasses renouvelables, notamment par la méthanisation, qui représente à la fois une voie de valorisation des déchets, résidus, et coproduits agricoles, industriels ou urbains, et un moyen de production d'une énergie renouvelable.

Le processus de méthanisation de matières organiques par des populations de microorganismes permet d'aboutir à la production, d'une part, d'un digestat, représentant une alternative aux engrais chimiques, et, d'autre part, de biogaz. Ce dernier est composé notamment de méthane CH₄, dans une proportion variant d'une méthanisation à l'autre, tout en étant généralement comprise entre 50 et 70% en volume. La proportion de dioxyde de carbone CO₂ retrouvée dans le biogaz, également variable, est comprise, quant à elle, entre 25 à 50% en volume, par rapport au volume total du biogaz produit. Enfin, le biogaz comporte des composés minoritaires, comme du sulfure d'hydrogène H₂S, de l'ammoniac NH₃, et autres gaz.

Une fois les impuretés et les gaz considérés comme indésirables, notamment le CO₂ et l'H₂S, éliminés du biogaz, on obtient alors du « biométhane » qui peut être injecté directement dans les réseaux existants, puis être utilisé pour les usages domestiques et industriels, ou qui peut être employé en tant que carburant automobile.

Une récente étude, commandée par l'ADEME (Agence De l'Environnement et de la Maitrise de l'Energie), montre qu'à l'horizon 2030, les besoins de stockage des excédents d'électricité pourraient être couverts par la mobilisation du CO₂ contenu dans le biogaz produit par les unités de méthanisation raccordées aux réseaux.

En effet, la méthanation d'H₂, produit électrochimiquement à partir d'électricité, et injectée dans des unités de méthanisation, au sein desquelles est produit du CO₂, permettrait ainsi de presque doubler la quantité de biométhane injectable dans le réseau de distribution, tout en mobilisant ledit CO₂ produit par la méthanisation, au coût marginal, et en mutualisant les infrastructures de conditionnement et d'injection du gaz.

Également, d'autres travaux ont été conduits sur la fermentation du syngas, composé essentiellement de monoxyde de carbone CO et de dihydrogène H₂ pour la production d'intermédiaires chimiques. Une équipe scientifique a notamment démontré la faisabilité de la production biologique de méthane à partir de syngas (Youngsukkasem et al., 2016).

La production d'hydrogène H₂ issu de la gazéification de la biomasse nécessite une étape d'épuration pour atteindre la qualité pile à combustible de l'hydrogène H₂ pour laquelle le coût semble très élevé. La fermentation du syngas en méthane CH₄ représente, par conséquent, une alternative intéressante et économique à l'épuration et la valorisation du syngas.

La combinaison de ces traitements avec un méthaniseur raccordé aux réseaux de distribution de gaz permettrait également d'améliorer la valorisation des syngas en limitant les investissements de purification et en mutualisant les postes d'injection de gaz.

Pour en revenir à présent à la méthanisation de la matière organique, il est à noter que, à la fin du processus de transformation anaérobie de cette matière qui conduit à la production de méthane, cohabitent deux voies de production de méthane : la voie acétotrophe, à partir de l'acétate CH₃COOH, et la voie hydrogénotrophe de production de CH₄ à partir de CO₂ et H₂. Il est ainsi estimé que cette dernière voie, dite hydrogénotrophe, représente généralement de l'ordre de 25 % du méthane CH₄ produit par un méthaniseur.

Les voies métaboliques de la méthanation du CO₂ et de l'hydrogène H₂ sont donc connues, ainsi que les microorganismes impliqués.

Il a également été montré que des microorganismes anaérobies du même type peuvent convertir le monoxyde de carbone CO et l'hydrogène H₂ en méthane CH₄.

La réaction globale de la voie hydrogénotrophe de production du méthane CH₄ à partir de dioxyde de carbone CO₂ ou réaction de méthanation, peut être écrite comme suit : CO₂ + 4 H₂ → CH₄ + 2 H₂O.

Enfin, la réaction globale de production de méthane CH₄ à partir de monoxyde de carbone CO, ou réaction de méthanation du monoxyde de carbone, peut être écrite comme suit : CO + 3 H₂ → CH₄ + H₂O.

Ces voies métaboliques de méthanation biologique sont connues et étudiées depuis les années 70 (Wise et al., 1978).

Cependant, l'application industrielle de la méthanation fait face à deux principaux freins technologiques.

Le premier frein technologique concerne la dissolution, dans le milieu de culture liquide, de l'hydrogène H₂ et/ou du monoxyde de carbone CO, cette dissolution étant nécessaire à l'assimilation de ces gaz par les microorganismes. En effet, ces deux gaz sont considérés comme étant des gaz qui sont peu solubles, et en particulier beaucoup moins solubles que le dioxyde de carbone. D'autre part, ces gaz (CO₂, CO, H₂) ne doivent pas, ou très peu, se retrouver dans le gaz produit (méthane CH₄) en vue d'une injection dans un réseau de distribution.

Le deuxième frein technologique est lié à la culture et à la maitrise du consortium de microorganismes hydrogénotrophes qui permettent la méthanation du dioxyde de carbone CO₂, ou du monoxyde de carbone CO, avec du dihydrogène H₂.

Dans l'état de la technique, on connait notamment les demandes internationales publiées sous les numéros WO 2019/034820 et WO 2019/034819 qui décrivent un réacteur de méthanation biologique, pour une production de gaz riche en méthane de synthèse, par conversion biologique de dioxyde de carbone CO₂ et de dihydrogène H₂.

Les réacteurs décrits dans ces demandes internationales comportent plus particulièrement une enceinte dans laquelle on retrouve, en partie basse, une entrée primaire d'eau, une entrée de dihydrogène H₂ et une entrée de dioxyde de carbone CO₂ et, en partie haute, une sortie pour le méthane de synthèse CH₄ et une sortie pour l'eau.

Les réacteurs comportent également un matériau support pour former un lit de flore méthanogène, qui permet de concentrer la flore méthanogène et a pour objet d'améliorer la surface de contact entre les bactéries et les réactifs afin d'améliorer les rendements de conversion du CO₂ et du H₂ en CH₄.

Ainsi, dans les réacteurs qui sont proposés dans ces deux demandes, la flore hydrogénotrophe se développe sur le lit fixé à travers lequel passe le gaz à fermenter.

Cependant, l'inconvénient principal de tels dispositifs réside dans le fait que le lit fixé doit être suffisamment dense pour permettre aux gaz réactifs (notamment le H₂, peu soluble) de se dissoudre complètement et d'être consommés par les microorganismes (Bassani et al., 2016).

En outre, il est à noter également que le volume du lit fixé doit être suffisamment important.

Dans la publication scientifique susmentionnée, Bassani et al. ont montré qu'il était possible de convertir 8,2 V H₂/V_{lit fixé}/Jour, pour une production de méthane liée à la méthanation de 2,1 V CH₄/V_{lit fixé}/Jour.

Cependant, un réacteur complémentaire de méthanation d'un volume représentant au moins 25 % du volume du réacteur de méthanisation était nécessaire dans leur étude.

Par ailleurs, avec cette technologie de diffusion du gaz sous un lit fixé, de l'hydrogène H₂ reste inévitablement présent dans le biométhane CH₄ obtenu. En conséquence, celui-ci ne peut donc pas être injecté directement dans le réseau de gaz naturel.

D'autres études ont démontré qu'une succession de bioréacteurs à lit fixés est nécessaire pour atteindre un taux de méthane CH₄ suffisant pour l'injection (Alitalo et al., 2015).

Toutefois, une accumulation de bioréacteurs a pour effet d'augmenter substantiellement le coût d'une telle installation, tout en complexifiant la maitrise de celle-ci.

De plus, le temps de démarrage d'un bioréacteur à lit fixé est généralement long car le biofilm doit se former et se fixer à la surface du support.

Ainsi, dans une étude publiée en 2015 et menée avec un système à lit fixé non immergé, « trickle bed reactor » (réacteur à lit à écoulement), Burkhardt obtient un biométhane à 98% de CH₄ avec une production spécifique de 1,5 V CH₄/V_{lit fixé}/Jour.

Une deuxième technologie, utilisée pour la méthanation biologique, prévoit l'utilisation de membranes d'injection de gaz servant alors de support de fixation pour les microorganismes hydrogénotrophes.

La demande de brevet européen publiée sous le numéro EP 3 418 371 traite ainsi de la méthanation du syngas, par la mise en oeuvre d'une installation comprenant :
- une unité pour la pyrolyse / gazéification, configurée pour la réception de matériel organique et la génération de syngaz à partir de ce matériel ;
- une membrane configurée pour être positionnée à l'intérieur d'un milieu liquide comprenant une population microbienne, la membrane comprenant au moins une fibre creuse autour de laquelle un biofilm microbien est susceptible de se former pour faciliter la conversion du syngas en méthane.

On retrouve également, dans le brevet européen délivré sous le numéro EP 2 771 472, et traitant la méthanation du CO₂ et de l'H₂, un procédé pour augmenter la teneur en CH₄ dans un biogaz, dans lequel le bioréacteur peut incorporer un système d'injection comprenant des fibres creuses pour y introduire le H₂ et le biogaz.

La demande de brevet américain publiée sous le numéro US 2019/309256 décrit également un système permettant d'augmenter la teneur en méthane d'un biogaz, comprenant un réacteur de biogaz, un réacteur à membrane à biofilm de microorganismes méthanogènes, conçu pour convertir le dioxyde de carbone CO₂ et le H₂ en méthane CH₄.

Cependant, cette technologie présente l'inconvénient de nécessiter des surfaces de membranes importantes.

En effet, cette surface de membrane détermine la surface du biofilm qui s'y forme et donc la densité en microorganismes hydrogénotrophes. En effet, le taux de transfert du gaz dans le cas du procédé décrit dans le brevet européen EP 2 771 472 est de 15 NL H₂/jour/m².

De plus, il a été mis en évidence que le biofilm formé sur les membranes limite le transfert du gaz.

Ces facteurs font que le taux de production de gaz est généralement relativement faible : de l'ordre de 0.9 V/V/jour pour le procédé décrit dans le brevet européen EP 2 771 472.

En outre, tout comme pour les réacteurs à lit fixé, le temps de formation du biofilm au démarrage du procédé est généralement long.

Dans la demande de brevet français publiée sous le numéro FR 2 601 690, de bons résultats ont pu être obtenus par la mise au point d'un procédé de production de méthane par culture de *Methanobacterium thermoautotrophicum* avec un procédé à lit fixé équipé d'une turbine d'agitation rapide disposée en dessous du garnissage pour obtenir la dispersion du gaz.

Cependant, il a été montré que l'énergie demandée dans le cas de l'utilisation de turbine seule pour la fermentation de gaz ne pouvait être rentable économiquement car les ratio énergie-volume sont trop importants (Bredwell et al., 2008). Ces procédés à l'étude en infiniment mélangé permettent toutefois de montrer qu'il est possible de réaliser la méthanation biologique avec des taux de production en méthane CH₄ importants, allant de 250 V/V/jour pour le procédé décrit dans la demande FR 2 601 690 et pouvant aller, pour d'autres procédés mis au point utilisant l'infiniment mélangé, jusqu'à 500 V/V/jour (Seifert et al., 2014).

Ainsi, certains des procédés actuellement proposés dans l'état de la technique sont basés sur la mise en oeuvre d'une flore microbienne sélectionnée, qui n'est pas toujours facilement accessible, et d'une alimentation de cette flore par un substrat nutritif synthétique dont la formulation est complexe à définir.

La présente invention se veut à même de remédier, au moins en partie, aux inconvénients des dispositifs connus dans l'état de la technique.

Dans une démarche inventive, il a été imaginé de développer un procédé de méthanation biologique et en continu de dihydrogène H₂ et d'un gaz choisi parmi le dioxyde de carbone CO₂ et le monoxyde de carbone CO, en vue de la production de biométhane CH₄ ou d'un biogaz riche en méthane CH₄, dans un bioréacteur anaérobie comprenant un milieu réactionnel, contenant au moins un substrat liquide avec des matières organiques à dégrader, et une flore microbienne, comprenant au moins des microorganismes méthanogènes hydrogénotrophes, apte à catalyser la transformation de H₂ et de CO₂ ou de H₂ et de CO en CH₄, ledit procédé étant caractérisé en ce qu'il comprend, au moins, les étapes suivantes :
a) on alimente, en continu, ledit bioréacteur anaérobie avec ledit substrat liquide ;
b) on filtre, en continu, ledit milieu réactionnel au travers d'une membrane de filtration présentant un seuil de coupure compris entre 0,01 µm et 0,2 µm, apte à retenir une partie du milieu réactionnel contenant la flore microbienne et les macromolécules présentant une taille supérieure audit seuil de coupure, et on évacue un digestat ;
c) on injecte, en continu, au sein dudit milieu réactionnel que comporte ledit bioréacteur anaérobie, au moins un premier gaz consistant en du dihydrogène H₂ ou en du syngas, ce dernier comprenant au moins du monoxyde de carbone CO et du dihydrogène H₂, et, optionnellement, un deuxième gaz choisi parmi le monoxyde de carbone CO, le dioxyde de carbone CO₂ et le biogaz, ledit au moins un gaz étant injecté dans ledit milieu réactionnel au travers d'un contacteur membranaire comprenant une membrane microporeuse hydrophobe, l'injection dudit au moins un gaz au sein dudit milieu réactionnel étant effectuée à une pression P1 supérieure à la pression limite Pl de formation des bulles dudit au moins gaz dans un liquide ;
d) on récupère du biométhane CH₄, ou un biogaz riche en méthane CH₄, produit au sein dudit bioréacteur anaérobie.

Dans un exemple de réalisation avantageux, on effectue l'étape b) de filtration du milieu réactionnel et/ou l'étape c) d'injection d'au moins un gaz au sein du milieu réactionnel au moyen d'une boucle de recirculation extérieure audit bioréacteur anaérobie.

Dans un autre exemple de réalisation, tout aussi avantageux, on effectue l'étape b) de filtration du milieu réactionnel et/ou l'étape c) d'injection d'au moins un gaz au sein du milieu réactionnel, dans le bioréacteur anaérobie.

Tout préférentiellement, on injecte ledit au moins un gaz au sein dudit milieu réactionnel au cours de l'étape c) à une pression P1 supérieure d'au moins 10 mbar à la pression limite Pl de formation des bulles de gaz dans un liquide.

Par exemple, on injecte ledit au moins un gaz au sein dudit milieu réactionnel au cours de l'étape c) à une pression P1 comprise entre PI+10 mbars et PI+500 mbars.

Préférentiellement, on injecte, au cours de l'étape c), un premier gaz consistant en du dihydrogène H₂ et un deuxième gaz consistant en du biogaz, et en ce que l'on injecte au moins quatre moles de dihydrogène H₂ pour une mole de dioxyde de carbone CO₂ contenu dans le biogaz injecté.

On peut également injecter, au cours de l'étape c), un premier gaz consistant en du syngas.

Dans une première variante de réalisation du procédé de l'invention, au cours de l'étape a), on alimente, en continu, ledit bioréacteur anaérobie avec un substrat liquide consistant en un effluent de méthaniseur.

Dans une deuxième variante de réalisation, ledit bioréacteur anaérobie consiste en un méthaniseur et, au cours de l'étape a), on alimente, en continu, ledit méthaniseur avec un substrat liquide consistant en un effluent industriel et/ou en un effluent agricole et/ou un effluent issu d'assainissement des eaux de station d'épuration.

Dans cette deuxième variante, il est envisageable d'injecter dans ledit méthaniseur, au cours de l'étape c), uniquement un premier gaz consistant en du dihydrogène H₂ ou en du syngas.

La présente invention est également relative à une installation pour la mise en oeuvre du procédé de l'invention de méthanation biologique et en continu de dihydrogène H₂ et d'un gaz choisi parmi le dioxyde de carbone CO₂ et le monoxyde de carbone CO, en vue de la production d'un biogaz riche en méthane CH₄.

L'installation comporte, au moins, un bioréacteur anaérobie comprenant un milieu réactionnel, contenant au moins un substrat liquide avec des matières organiques à dégrader, et une flore microbienne comprenant au moins des microorganismes méthanogènes hydrogénotrophes, aptes à catalyser la transformation de H₂ et de CO₂ ou de H₂ et de CO en CH₄.

Ladite installation comporte également au moins des moyens d'alimentation dudit bioréacteur anaérobie en substrat liquide, des moyens d'extraction du biogaz riche en méthane CH₄ produit, et un module de filtration dudit milieu réactionnel au travers d'une membrane de filtration que comporte ledit module.

Cette membrane présente un seuil de coupure compris entre 0,01 et 0,2 µm, apte à retenir une partie du milieu réactionnel contenant la flore microbienne et des macromolécules présentant une taille supérieure audit seuil de coupure, et à laisser passer un digestat, et elle est reliée à des moyens d'évacuation dudit digestat.

Ladite installation comporte encore des premiers moyens d'injection, au sein dudit milieu réactionnel que comporte ledit bioréacteur anaérobie, d'au moins un premier gaz consistant en du dihydrogène H₂ ou en du syngas, et, optionnellement, des deuxièmes moyens d'injection d'un deuxième gaz choisi parmi le monoxyde de carbone CO, le dioxyde de carbone CO₂ et le biogaz.

Le(s)dit(s) moyen(s) d'injection comportent, d'une part, un contacteur membranaire comprenant une membrane microporeuse hydrophobe et, d'autre part des moyens de contrôle et de maintien de la pression d'injection P1 dudit au moins un premier gaz au sein dudit milieu réactionnel à une pression supérieure à la pression limite Pl de formation des bulles de gaz injecté dans un liquide.

Le procédé selon l'invention et tel que décrit précédemment présente de nombreux avantages et permet de remédier à de nombreuses problématiques, jusque-là non résolues, que présentaient les procédés et dispositifs de méthanation proposés dans l'état de la technique.

Dans un premier temps, le procédé ne nécessite pas la mise en culture d'une flore microbienne sélectionnée et très spécifique, pour laquelle le maintien d'une croissance optimale, nécessitant un milieu nutritif bien particulier, peut s'avérer extrêmement complexe. Le présent procédé met en oeuvre, dans un bioréacteur anaérobie de méthanation, une flore que l'on peut qualifier de « sauvage », présente dans tous réacteurs de méthanisation. Cette simplicité d'accès aux souches garantit la sécurité de fonctionnement du présent système de méthanation.

Deuxièmement, le procédé de l'invention permet une maîtrise extrêmement fine de l'injection de gaz dans un réacteur anaérobie de méthanation et, par conséquent, dans le milieu réactionnel de fermentation. Or, un tel contrôle de l'injection de gaz est primordial pour une amélioration de l'efficacité du procédé de méthanation, notamment car les gaz que sont l'hydrogène H₂ et le monoxyde de carbone CO sont des inhibiteurs de la méthanisation.

En outre, par un contrôle de l'injection de ces gaz réactifs, on limite, de manière substantielle, la teneur en hydrogène H₂/monoxyde de carbone CO/ dioxyde de carbone CO₂, au sein du gaz méthane CH₄ produit, en sorte que les spécifications définies par l'exploitant du réseau de distribution, pour injecter le méthane CH₄ obtenu dans ledit réseau, soient respectées.

Enfin, le procédé objet de la présente invention permet de limiter l'énergie nécessaire à la dissolution des gaz peu solubles (CO et H₂) dans le milieu nutritif.

A noter également que la mise en oeuvre du procédé de méthanation selon la présente invention permet de conserver une haute densité de microorganismes actifs dans le bioréacteur anaérobie de méthanation.

D'autres buts et avantages de la présente invention pourront apparaitre au cours de la description qui va suivre, qui se rapporte à des modes de réalisation qui ne sont donnés qu'à titre d'exemples indicatifs et non limitatifs.

La compréhension de cette description sera facilitée en se référant aux dessins joints en annexe et dans lesquels :
[Fig.1] représente de manière schématique, un premier mode de réalisation d'une installation pour la mise en oeuvre du procédé de méthanation selon la présente invention ;
[Fig.2] représente de manière schématique, un deuxième mode de réalisation d'une installation pour la mise en oeuvre du procédé de méthanation selon la présente invention.

En référence à ces dessins, le procédé de l'invention consiste, plus particulièrement en un procédé de méthanation, par voie biologique et en continu, d'hydrogène H₂ avec un gaz consistant soit en du dioxyde de carbone CO₂, soit en du monoxyde de carbone CO, selon les réactions de méthanation dont les équations globales ont été rappelées en introduction, à savoir :

CO₂ + 4 H₂ → CH₄ + 2 H₂O ;

CO + 3 H₂ → CH₄ + H₂O.

Le procédé de la présente invention peut également consister en un procédé de méthanation biologique et en continu d'hydrogène H₂ et avec, à la fois, du dioxyde de carbone CO₂ et du monoxyde de carbone CO, comme cela sera explicité plus en détails ci-après au travers d'exemples de gaz pouvant être utilisés dans ce procédé de méthanation.

Par la mise en oeuvre du procédé de méthanation selon l'invention, on peut obtenir du biométhane CH₄ ou un gaz riche en biométhane CH₄, c'est-à-dire comportant une proportion (V/V) de CH₄ supérieure ou égale 90%, de préférence supérieure ou égale à 95%, et, plus préférentiellement encore, supérieure ou égale à 97%, notamment lorsque les gaz de départ consistent en du dihydrogène H₂ et de dioxyde de carbone CO₂.

Le méthane CH₄ produit ainsi, à partir d'énergies renouvelables, peut avantageusement être injecté dans un réseau existant de transport et de distribution de gaz naturel.

A noter que, lorsque la réaction de méthanation mise en oeuvre dans le procédé de l'invention implique, en tant que gaz de départ, du monoxyde de carbone CO et du dihydrogène H₂, le gaz obtenu comporte une proportion (V/V) comprise entre 50 et 60% de CH₄. Aussi, l'injection de celui-ci dans un réseau existant pourra nécessiter la conduite d'une étape d'épuration éventuelle.

Le procédé de la présente invention est mis en oeuvre dans un bioréacteur anaérobie 1 au sein duquel est mis en place un milieu réactionnel 2 apte à permettre le déroulement de la réaction de méthanation du dioxyde de carbone CO₂ et/ou de la réaction de méthanation du monoxyde de carbone CO.

A cet effet, ledit milieu réactionnel 2 est composé, d'une part, d'un substrat liquide 3 comprenant au moins des matières organiques à dégrader et, d'autre part, une flore microbienne comprenant au moins des microorganismes méthanogènes hydrogénotrophes. En effet, ces derniers sont aptes à catalyser la transformation de H₂ et de CO₂ ou de H₂ et de CO en CH₄.

Le procédé de méthanation biologique objet de la présente invention est mis en oeuvre selon un fonctionnement en continu au sein du bioréacteur anaérobie 1.

En d'autres termes, et bien qu'une certaine séquentialité des étapes dudit procédé de méthanation puisse être évoquée dans la suite de la description, il convient de considérer que, selon la présente invention, ces différentes étapes, dans le procédé de l'invention, sont mises en oeuvre en série et fonctionnent simultanément et en continu.

Dans une étape, notée étape a), du procédé de méthanation objet de la présente invention, on alimente, en continu, le bioréacteur anaérobie 1 dans lequel se déroule le procédé avec au moins un substrat liquide 3.

Ledit substrat liquide 3 vient donc s'ajouter en continu au milieu réactionnel 2 qui est présent au sein du bioréacteur anaérobie 1.

Dans une étape notée étape b) du procédé selon l'invention, on filtre, là encore de manière continue, ledit milieu réactionnel 2 au travers d'une membrane de filtration 4 que comporte un module de filtration 40.

Cette membrane de filtration 4 présente un seuil de coupure compris entre 0,01µm et 0,2 µm.

Une membrane d'ultrafiltration présentant un seuil de coupure comprise entre 20 et 50 kDa peut être utilisée dans le module de filtration 40.

De tels seuils de coupure sont particulièrement intéressants, car ils permettent une rétention d'une partie du milieu réactionnel 2, contenant la flore active, ainsi que certaines macromolécules organiques, présentant une taille supérieure audit seuil de coupure, et qui sont susceptibles de nécessiter un temps important pour aboutir à leur dégradation par les microorganismes.

Ces molécules sont, par conséquent, réacheminées, avec une partie du milieu réactionnel 2, vers le bioréacteur anaérobie 1, afin de poursuivre leur dégradation par la flore microbienne, tandis que cette étape de filtration permet d'évacuer, de l'autre côté de la membrane de filtration 4, un digestat 5. Ce dernier contient les molécules présentant une taille inférieure au seuil de coupure de ladite membrane 4, et qui ont donc été dégradées de manière satisfaisante par les microorganismes présents dans le bioréacteur anaérobie 1.

La mise en oeuvre, dans le procédé de méthanation de l'invention, d'une telle étape b) de filtration, présente l'avantage de permettre une conservation d'une biomasse active, et à haute densité, supérieure à 5g/L de MVES (Matières Volatiles En Suspension), de préférence de l'ordre de 7g/L de MVES, dans le bioréacteur anaérobie 1 de méthanation. Cette biomasse active est constituée par les microorganismes qui dégradent le substrat pour leur croissance et leur multiplication et catalysent la réaction de méthanation.

La filtration, au travers de la membrane 4, cette dernière constituant une barrière physique, permet notamment d'assurer un temps de séjour plus long pour les macromolécules à dégradation plus lente.

On conserve également ainsi, dans le bioréacteur anaérobie 1, une biomasse libre qui est plus active, et par conséquent plus efficace, qu'une biomasse fixée, sous la forme de biofilms, notamment au travers de lits de billes ou de membranes.

La mise en oeuvre d'une telle étape de filtration, et la conservation d'une biomasse, notamment d'une flore libre, qui en découle, permet également, de manière particulièrement intéressante, de réduire le temps de démarrage du procédé de méthanation de l'invention, en comparaison avec la mise en oeuvre d'une technologie à lit fixé ou à biofilm.

En effet, pour la formation, au niveau d'un support quelconque (billes, membranes ou autres) d'un biofilm actif qui soit efficace, un temps certain est nécessaire, et le présent procédé permet de s'affranchir, par le maintien d'une biomasse active libre, de cette contrainte.

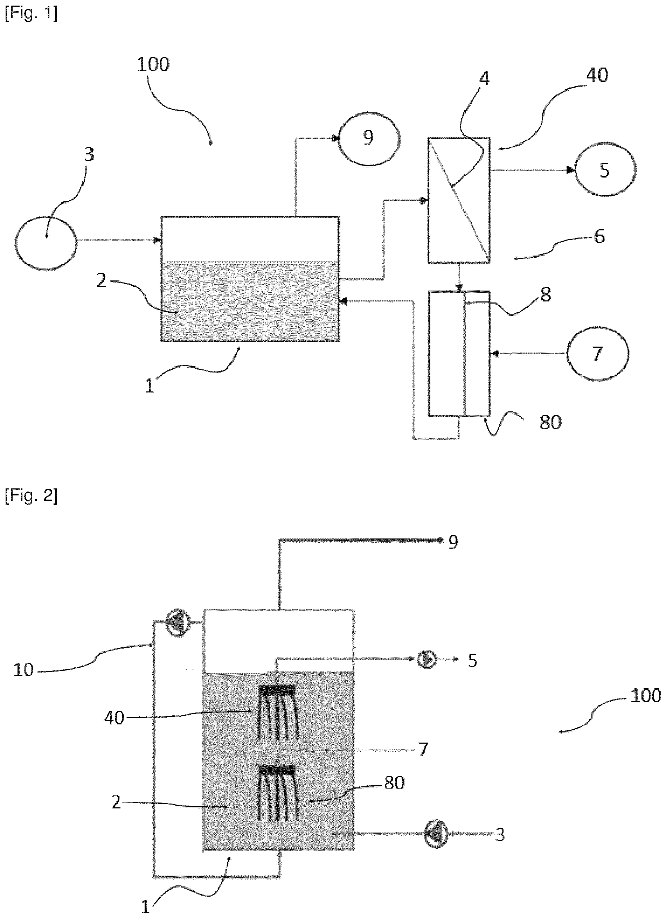

En ce qui concerne la mise en oeuvre de l'étape b) de filtration, celle-ci peut être par exemple effectuée au moyen d'une boucle de recirculation 6 extérieure au bioréacteur anaérobie 1, et au niveau de laquelle est acheminé le milieu réactionnel 2 qu'il convient de filtrer, par exemple au moyen d'une pompe. Ce mode de réalisation particulier et non limitatif est illustré sur la figure 1, la pompe n'étant toutefois pas représentée.

En effet, il est également envisageable que cette étape b) de filtration du milieu réactionnel soit mise en oeuvre au sein même du milieu réactionnel 2, dans le bioréacteur anaérobie de méthanation 1, comme représenté sur la figure 2 jointe.

Dans ce cas particulier, le module de filtration 40 incorporant au moins une membrane de filtration 4 est immergée dans le milieu réactionnel 2 du bioréacteur 1 et une pompe de succion permet la filtration dudit milieu réactionnel 2.

Dans une autre étape particulière et essentielle au procédé de méthanation de l'invention, notée étape c), on procède à une injection, toujours de manière continue, d'au moins un gaz 7 au sein du milieu réactionnel 2 que comporte le réacteur anaérobie 1 au sein duquel se déroule ledit procédé.

Ledit au moins un gaz 7 est injecté au sein dudit milieu réactionnel 2 par le biais d'une membrane microporeuse hydrophobe 8 que comporte un contacteur membranaire 80.

Plus spécifiquement, un contacteur membranaire 80, tel que celui mis en oeuvre dans le procédé objet de la présente invention, est constitué d'un faisceau de fibres creuses microporeuses et réalisées en un matériau hydrophobe, et faisant office de membrane microporeuse hydrophobe 8.

Ledit au moins un gaz 7 qu'il convient d'injecter dans le milieu réactionnel 2 circule à l'intérieur desdites fibres creuses microporeuses, tandis que ledit milieu réactionnel 2 circule à l'extérieur.

Il est également envisageable que ledit au moins un gaz 7 circule à l'extérieure des fibres creuses microporeuses de ladite membrane microporeuse hydrophobe 8 tandis que le milieu réactionnel 8 circule, lui, à l'intérieur des fibres, en particulier lorsque le gaz est injecté dans le milieu réactionnel 2 au moyen d'une boucle de recirculation 6 extérieure, comme cela sera développé plus en détails dans la suite de la description.

Ce type de module de contacteur membranaire présente une très grande surface spécifique, et en particulier bien plus importante en comparaison à la surface spécifique d'une colonne à garnissage.

A noter également que différentes géométries de contacteurs membranaires peuvent être envisageables.

Selon le procédé de l'invention, on effectue une telle injection dudit au moins un gaz 7 au sein dudit milieu réactionnel 2 étant effectuée à une pression P1 supérieure à la pression limite Pl de formation des bulles dudit au moins gaz dans un liquide.

D'une part, il est à noter que la maitrise de la pression P1 à laquelle ledit au moins un gaz 7 est injecté permet, de manière particulièrement avantageuse, de contrôler finement la concentration dudit gaz 7 qui se dissout dans le milieu réactionnel 2.

D'autre part, une injection dudit au moins un gaz 7 à une pression P1 supérieure à la pression limite Pl de formation des bulles dudit gaz 7 permet d'empêcher la formation d'un biofilm au niveau de la surface de la membrane microporeuse hydrophobe 8. Or, la formation d'un tel biofilm aurait pour conséquence de diminuer le transfert du gaz 7 à injecter dans la phase liquide, constitué par le milieu réactionnel 2.

Par conséquent, le fait de maintenir, dans l'étape c) du présent procédé, ledit au moins un gaz 7 à injecter à une pression P1 supérieure à la pression limite Pl de formation des bulles de ce gaz 7 dans un liquide permet ainsi de fonctionner avec une surface de membrane 8 substantiellement moins importante que la surface qui est nécessaire lorsque sont mises en oeuvre des membranes sur lesquelles se forment un biofilm.

Dans l'étape c) selon la présente invention, on injecte en continu, au sein du milieu réactionnel 2, au moins un premier gaz 7 qui consiste, par exemple, soit en du dihydrogène H₂, soit en du syngas. Ce dernier est composé d'un mélange de gaz comprenant essentiellement du monoxyde de carbone CO et du dihydrogène H₂. Il peut également comporter une certaine fraction de dioxyde de carbone CO₂.

En effet, dans une première variante de réalisation du procédé de méthanation de l'invention, correspondant à une méthanation *in situ,* ledit bioréacteur anaérobie 1 consiste en un méthaniseur au sein duquel est produit du dioxyde de carbone CO₂.

Plus précisément, dans cette variante de réalisation, au cours de l'étape a), on alimente, en continu, ledit méthaniseur avec un substrat liquide 3 consistant, par exemple mais non limitativement, en un effluent industriel et/ou en un effluent agricole et/ou en un effluent issu d'assainissement des eaux de station d'épuration (STEP) des eaux usées, constitué de matières organiques, qui vont être méthanisées directement au sein du méthaniseur par des microorganismes, et aboutir à la production de biogaz comprenant une certaine fraction de dioxyde de carbone CO₂.

Ainsi, dans cette première variante de réalisation, il est envisageable de procéder uniquement à une injection, au cours de l'étape c) du procédé de l'invention, au sein du milieu réactionnel 2, de dihydrogène H₂. Celui-ci sera combiné avec le dioxyde de carbone CO₂ produit par méthanisation dans le bioréacteur anaérobie 1, consistant alors en un méthaniseur, et transformé en méthane CH₄ par le biais d'une réaction de méthanation catalysée par les microorganismes méthanogènes hydrogénotrophes présents dans ledit méthaniseur.

Dans une seconde variante de réalisation du procédé de méthanation de l'invention, correspondant, cette fois ci, à une méthanation *ex situ,* ledit bioréacteur anaérobie 1 consiste en un réacteur anaérobie secondaire qui est relié, au travers de moyens adaptés, à un méthaniseur.

Dans ce cas de figure particulier, le bioréacteur anaérobie 1 est alimenté en continu, au cours de l'étape a), avec un substrat liquide 3 consistant en un effluent de méthaniseur.

Le fait d'alimenter le bioréacteur anaérobie 1 avec un effluent de méthaniseur présente plusieurs avantages.

D'une part, l'alimentation d'un réacteur secondaire spécifique, en l'occurrence le bioréacteur anaérobie 1, avec un effluent de méthaniseur entraîne un apport d'éléments nutritifs nécessaires au développement d'une biomasse active.

D'autre part, on permet par ce biais un ensemencement dudit bioréacteur 1 avec la flore microbienne existant préalablement dans ledit méthaniseur et comportant des microorganismes méthanogènes hydrogénotrophes aptes à catalyser une réaction de méthanisation, et donc de méthanation.

On dispose par conséquent immédiatement, dès le démarrage du procédé, de microorganismes actifs pour la production de méthane à partir notamment d'hydrogène H₂ et de gaz carbonique CO ou CO₂, ou à partir de syngas, et on maitrise aisément la conduite de ce consortium microbien impliqué dans cette fermentation.

Aussi, il ressort clairement de ce qui précède que la mise en oeuvre de cette variante de réalisation du procédé de l'invention implique nécessairement l'injection, au cours de l'étape c), d'un premier gaz comprenant au moins du dihydrogène H₂ et un oxyde de carbone CO ou CO₂.

En effet, contrairement à la première variante de réalisation où du dioxyde de carbone CO₂ était produit directement par méthanisation dans le bioréacteur anaérobie 1, tel n'est pas le cas dans la seconde variante. Il convient, par conséquent, d'introduire, au sein dudit bioréacteur, en plus du dihydrogène H₂, au moins un oxyde de carbone.

Aussi, il est envisageable d'injecter au sein dudit milieu réactionnel 2, au cours de l'étape c) du procédé, en tant que premier gaz, du syngas qui comprend au moins du monoxyde de carbone CO et du dihydrogène H₂, et qui peut également comporter, généralement en plus faible teneur, du dioxyde de carbone CO₂.

On peut également, au cours de cette étape c), et de manière optionnelle, quelle que soit la variante de réalisation retenue, injecter un deuxième gaz.

Celui-ci peut être préférentiellement choisi parmi le monoxyde de carbone CO, le dioxyde de carbone CO₂ et le biogaz, ce dernier comportant principalement un mélange de dioxyde de carbone CO₂ et de méthane CH₄.

Ainsi, par exemple, il est envisageable que l'on injecte, au cours de l'étape c), en tant que premier gaz, du dihydrogène H₂ et, en tant que deuxième gaz, du biogaz.

Dans cet exemple, tout préférentiellement, les débits d'injection de dihydrogène H₂ et de dioxyde de carbone CO₂ contenu dans le biogaz seront contrôlés de façon à respecter la stoechiométrie de la réaction de méthanation. En d'autres termes, il convient d'injecter ces gaz H₂ et CO₂ avec un ratio volumique ou molaire égal, ou sensiblement égale à 4/1 pour assurer une méthanation efficace.

Par exemple, les débits d'injection des différents gaz peuvent être contrôlés au moyen d'un débitmètre massique.

A cet effet, il est préférable de procéder à deux injections distinctes et indépendantes, au sein du milieu réactionnel 2, du dihydrogène H₂ d'un côté, et du biogaz de l'autre.

Également, compte tenu de la grande solubilité, dans un milieu liquide, du dioxyde de carbone CO₂, en comparaison avec celle du dihydrogène H₂, un dispositif plus simple, notamment un dispositif diffuseur ou un système de bullage, pourra être utilisé.

Dans cet exemple de réalisation, lorsque les gaz de départ sont du CO₂ et du H₂, le gaz produit par méthanation 9, et récupéré en sortie du réacteur anaérobie 1 au cours d'une étape d) du procédé, est composé majoritairement de méthane CH₄, dans une proportion (V/V) supérieure ou égale à 95 %.

Il a été évoqué, précédemment dans la description, en lien avec l'étape b) de filtration en continu du milieu réactionnel 2, que celle-ci pouvait être effectuée soit de manière déportée au bioréacteur anaérobie 1, au moyen d'une boucle de recirculation externe 6, soit au sein même du milieu réactionnel 2, à l'intérieur dudit bioréacteur anaérobie 1.

Il en est de même pour la mise en oeuvre de l'étape c) au cours de laquelle on injecte au moins un gaz 7 dans le milieu réactionnel 2.

En d'autres termes, l'étape c) d'injection de gaz 7 peut être réalisée au moyen d'une boucle de recirculation 6 extérieure au bioréacteur anaérobie 1 ou bien directement dans le milieu réactionnel 2, au sein dudit bioréacteur 1.

II est ainsi envisageable que l'étape b) de filtration en continu du milieu réactionnel 2 et que l'étape c) d'injection de gaz 7 soient effectuées en série au moyen d'une boucle de recirculation 6 extérieure au niveau de laquelle sont positionnés un module de filtration 40 et un contacteur à membrane 80.

Ces deux étapes peuvent également être mises en oeuvre toutes deux au sein même du bioréacteur anaérobie 1, en immergeant à la fois ledit module de filtration 40, permettant une évacuation du digestat 5 à l'extérieur dudit bioréacteur 1, ainsi que ledit contacteur à membrane 80, relié à une source externe d'au moins un gaz à injecter 7, au sein du milieu réactionnel 2, comme illustré sur la figure 2.

Dans ce dernier exemple de réalisation, en référence à la figure 2, le procédé de méthanation de l'invention peut intégrer une étape supplémentaire et optionnelle au cours de laquelle une partie du gaz produit par méthanation au sein du bioréacteur anaérobie 1 est renvoyé, au moyen d'une boucle de recirculation 10 au sein du milieu réactionnel 2.

En particulier, la recirculation du gaz est réalisée en cas d'utilisation de membrane de filtration 4 interne au bioréacteur 1 afin de limiter l'encrassement de celle-ci.

Il est également tout à fait possible que l'une de ces étapes, filtration sur membrane ou injection de gaz, soit mise en oeuvre intérieurement au bioréacteur anaérobie 1, tandis que l'autre est déportée à l'extérieur de celui-ci au moyen d'une boucle de recirculation 6.

Pour en revenir à présent à la pression P1 à laquelle on injecte ledit au moins un premier gaz 7 au sein dudit milieu réactionnel 2, il a déjà été établi préalablement dans la description que celle-ci devait être supérieure à la pression limite Pl de formation des bulles dudit gaz 7 dans un liquide.

De manière encore plus préférentielle, on injecte ledit au moins un gaz 7 au sein dudit milieu réactionnel 2 au cours de l'étape c) à une pression P1 qui est supérieure d'au moins 10 mbar à la pression limite Pl de formation des bulles de ce gaz 7dans un liquide.

A noter encore que, avantageusement, cette pression P1 d'injection du gaz 7 dans le milieu réactionnel 2 peut être comprise entre PI+10 mbars et PI+ 500 mbars.

Lorsque, au cours de l'étape c), on injecte un premier gaz 7 et un deuxième gaz au sein du milieu réactionnel 2, le premier gaz 7 injecté à une pression P1 supérieure à la pression limite Pl de formation des bulles de ce premier gaz 7 dans un liquide, tandis que le deuxième gaz est injecté à une pression P1' supérieure à la pression limite PI' de formation des bulles de ce deuxième gaz dans un liquide.

Ainsi, lorsque deux gaz sont injectés au cours de l'étape c), ils peuvent être soumis à des pressions d'injection P1 et P1' différentes selon leurs pressions limites respectives Pl et PI'.

La présente invention concerne également une installation 100 permettant la mise en oeuvre du procédé de méthanation de l'invention qui a été décrit précédemment dans le détail.

Cette installation 100 comprend notamment un bioréacteur anaérobie 1 se présentant préférentiellement sous la forme d'une enceinte tubulaire ou cylindrique, fermée et étanche, pour y conserver une atmosphère dépourvue, ou quasiment dépourvue, de dioxygène O₂ afin de conserver, à l'intérieur de l'enceinte, une anaérobiose propice aux réactions de méthanation des gaz.

Cette enceinte du bioréacteur anaérobie 1 est destinée à recevoir le milieu réactionnel méthanogène liquide 2 au sein duquel la réaction de méthanation peut s'effectuer, ce milieu étant constitué, d'une part, d'un substrat liquide 3 et d'une flore méthanogène, notamment comprenant des microorganismes méthanogènes hydrogénotrophes, aptes à catalyser la transformation de H₂ et de CO₂ ou de H₂ et de CO₂ en CH₄.

Au niveau de la paroi de ladite enceinte du bioréacteur 1 sont ménagées des ouvertures permettant de positionner des moyens d'arrivée des différents réactifs qui vont être nécessaires au bon déroulement des réactions de méthanation au sein du bioréacteur 1, ainsi que des moyens d'évacuation des produits issus de ces réactions.

Ainsi, l'installation 100 de l'invention comporte également, au moins, des moyens d'alimentation dudit bioréacteur anaérobie 1 en substrat liquide 3 ainsi que des moyens d'extraction du biogaz 9 riche en méthane CH₄ produit.

La présente installation 100 est également munie d'un module de filtration 40 dudit milieu réactionnel 2 au travers d'une membrane de filtration 4 que comporte ledit module 40.

Ce module de filtration 40 peut être externe au bioréacteur anaérobie 1, et positionné au sein d'une boucle de recirculation 6, ou bien être immergé dans le milieu réactionnel 2.

La membrane de filtration 4 de ce module 40 présente un seuil de coupure compris entre 0,01 et 0,2 µm. Ainsi, cette membrane 4 est apte à retenir une partie du milieu réactionnel 2 contenant notamment la flore microbienne ainsi que des macromolécules présentant une taille supérieure audit seuil de coupure, et à laisser passer un digestat 5 liquide, qui est évacué hors de l'installation 100. Des moyens d'évacuation du digestat 65 sont donc également prévus.

Une membrane d'ultrafiltration présentant un seuil de coupure compris entre 20 et 50 kDa peut équiper le module de filtration 40.

Ladite présente installation 100 de méthanation comporte encore des premiers moyens d'injection, au sein dudit milieu réactionnel 2 du bioréacteur anaérobie 1, d'au moins un premier gaz 7, consistant en du dihydrogène H₂ ou en du syngas.

Optionnellement, l'installation 100 peut également intégrer des deuxièmes moyens d'injection d'un deuxième gaz choisi parmi le monoxyde de carbone CO, le dioxyde de carbone CO₂ et le biogaz.

Les premiers moyens d'injection comportent, d'une part, un contacteur membranaire 80 comprenant une membrane microporeuse hydrophobe 8 et, d'autre part des moyens de contrôle et de maintien de la pression d'injection P1 dudit au moins un premier gaz au sein dudit milieu réactionnel 2 à une pression supérieure à la pression limite Pl de formation des bulles de ce premier gaz lorsqu'il est injecté dans un liquide.

Ces moyens de contrôle et de maintien de la pression d'injection à une pression déterminée peuvent être avantageusement constitués par des boucles de régulation de débit et de pression.

Lorsqu'ils sont présents au niveau de l'installation 100, les deuxièmes moyens d'injection d'un deuxième gaz peuvent être avantageusement similaires aux premiers moyens décrits d'injection du premier gaz 7, et se présenter sous la forme d'un contacteur membranaire.

Selon la solubilité du deuxième gaz qui peut être injecté dans le milieu réactionnel 2, ces deuxièmes moyens d'injection peuvent également être différents desdits premiers moyens d'injection du premier gaz 7.

Ces moyens peuvent alors consister en un dispositif de diffusion ou de bullage et participer ainsi à l'agitation du milieu, propice à l'amélioration des transferts gaz-liquide et à la limitation du colmatage des membranes de filtration.

L'enceinte du bioréacteur 1 comporte également des moyens d'évacuation du CH₄ produit par la réaction de méthanation, ces derniers étant avantageusement positionnés à proximité de l'extrémité haute du bioréacteur anaérobie 1 de méthanation.

A noter que de manière générale, et lorsque cela est applicable, les éléments et caractéristiques qui ont été décrits pour l'installation 100 de méthanation peuvent être appliqués au procédé de méthanation de l'invention, et inversement.

L'intérêt de la présente invention apparaitra encore plus clairement à la lecture de l'exemple non limitatif décrit ci-dessous.

### Exemple de réalisation du procédé de méthanation de l'invention :

Un procédé de méthanation « *in situ* » conforme à la présente invention a été mis en oeuvre dans un bioréacteur anaérobie 1 consistant en un méthaniseur et présentant un volume de 150 L.

Le bioréacteur anaérobie 1 utilisé aux fins de ce présent exemple fonctionne suivant le schéma représenté sur la figure 1 annexée.

Le substrat liquide 3, sous la forme d'un effluent industriel à 12 g/L de DCO (correspondant à la Demande Chimique en Oxygène) alimente en continu le bioréacteur anaérobie 1.

Le digestat 5 est éliminé également en continu à travers une membrane de filtration 4 présentant un seuil de coupure égal à 0,1µm, le module de filtration 40 incorporant ladite membrane 4 étant installé au niveau d'une boucle de recirculation 6 externe au bioréacteur anaérobie 1.

Un contacteur à membrane 80, pour l'injection de gaz dihydrogène H₂ au sein du milieu réactionnel 2, est installé sur la même boucle de recirculation 6, en série avec ledit module de filtration 40.

La quantité de dihydrogène H₂ injectée dans le milieu réactionnel 2 est ajustée, de façon stoechiométrique, à la quantité de CO₂ produite au sein du bioréacteur anaérobie 1 par la réaction de fermentation.

Le contrôle de la quantité d'hydrogène est effectué par un débitmètre massique thermique, le débitmètre ajustant la pression pour délivrer le bon débit. Le débit est ajusté à la qualité du gaz produit pour maintenir constant le taux CH₄-CO₂.

Ainsi, la pression P1 du dihydrogène H₂ à l'injection a été contrôlée, entre 0,6 et 2 bars, cette pression P1 étant supérieure à la pression limite Pl de formation des bulles d'hydrogène dans le milieu réactionnel 2.

Dans ces conditions, c'est-à-dire utilisant un effluent industriel à 12 g/L de DCO, avec un temps de séjour de 2,5 jours, les inventeurs ont pu produire un biométhane CH₄ présentant 97% de pureté, et contenant moins de 1% de dihydrogène H₂, tout en maintenant un rendement épuratoire supérieur à 90%.

Ainsi, il est à noter que l'injection de dihydrogène H₂ n'a pas eu d'influence défavorable sur les performances du réacteur anaérobie 1 consistant, ici, en un méthaniseur.

### Références :

Anni Alitalo, Marko Niskanen, Erkki Aura, Biocatalytic methanation of hydrogen and carbon dioxide in a fixed bed bioreactor, Bioresource Technology, Volume 196, 2015, Pages 600-605.

Ilaria Bassani, Panagiotis G. Kougias, Irini Angelidaki, In-situ biogas upgrading in thermophilic granular UASB reactor: key factors affecting the hydrogen mass transfer rate, Bioresource Technology, Volume 221, 2016, Pages 485-491.

Radeep Chaminda Munasinghe, Samir Kumar Khanal, Biomass-derived syngas fermentation into biofuels: Opportunities and challenges, Bioresource Technology, Volume 101, Issue 13, 2010, Pages 5013-5022.

A.H. Seifert, S. Rittmann, C. Herwig, Analysis of process related factors to increase volumetric productivity and quality of biomethane with Methanothermobacter marburgensis, Applied Energy, Volume 132, 2014, Pages 155-162.

Youngsukkasem, Supansa & Chandolias, Konstantinos & Taherzadeh, Mohammad. Syngas Biomethanation in a Semi-Continuous Reverse Membrane Bioreactor (RMBR). Fermentation, Volume 2, 2016, Page 8.

Bredwell MD, Srivastava P, Worden RM. Reactor Design Issues for Synthesis-Gas Fermentations. Biotechnology Progress, Volume 15, 1999, Pages 834-844.

D.L. Wise, C.L. Cooney, D.C. Augenstein, Biomethanation: anaérobie fermentation of CO2, H2 and CO to methane, Biotechnol. Bioeng. 20 (1978) 1153-1172. 1

## Revendications

1. Procédé de méthanation biologique et en continu de dihydrogène H₂ et d'un gaz choisi parmi le dioxyde de carbone CO₂ et le monoxyde de carbone CO, en vue de la production de biométhane CH₄ ou d'un biogaz riche en méthane CH₄, dans un bioréacteur anaérobie (1) comprenant un milieu réactionnel (2), contenant au moins un substrat liquide (3) avec des matières organiques à dégrader, et une flore microbienne, comprenant au moins des microorganismes méthanogènes hydrogénotrophes, apte à catalyser la transformation de H₂ et de CO₂ ou de H₂ et de CO en CH₄, ledit procédé étant **caractérisé en ce qu'**il comprend, au moins, les étapes suivantes :
a) On alimente, en continu, ledit bioréacteur anaérobie (1) avec ledit substrat liquide (3) ;
b) On filtre, en continu, ledit milieu réactionnel (2) au travers d'une membrane de filtration (4) présentant un seuil de coupure compris entre 0,01 µm et 0,2 µm, apte à retenir une partie du milieu réactionnel (2) contenant la flore microbienne et les macromolécules présentant une taille supérieure audit seuil de coupure, et on évacue un digestat (5) ;
c) On injecte, en continu, au sein dudit milieu réactionnel (2) que comporte ledit bioréacteur anaérobie (1), au moins un premier gaz (7) consistant en du dihydrogène H₂ ou en du syngas, ce dernier comprenant au moins du monoxyde de carbone CO et du dihydrogène H₂, et, optionnellement, un deuxième gaz choisi parmi le monoxyde de carbone CO, le dioxyde de carbone CO₂ et le biogaz, ledit au moins un gaz (7) étant injecté dans ledit milieu réactionnel (2) au travers d'un contacteur membranaire (80) comprenant une membrane microporeuse hydrophobe (8), l'injection dudit au moins un gaz (7) au sein dudit milieu réactionnel (2) étant effectuée à une pression P1 supérieure à la pression limite Pl de formation des bulles dudit au moins gaz (7) dans un liquide ;
d) On récupère du biométhane CH₄, ou un biogaz riche en méthane CH₄, produit au sein dudit bioréacteur anaérobie (1).

2. Procédé de méthanation selon la revendication 1 **caractérisé en ce que** l'on effectue l'étape b) de filtration du milieu réactionnel (2) et/ou l'étape c) d'injection d'au moins un gaz (7) au sein du milieu réactionnel au moyen d'une boucle de recirculation (6) extérieure audit bioréacteur anaérobie (1).

3. Procédé de méthanation selon la revendication 1 **caractérisé en ce que** l'on effectue l'étape b) de filtration du milieu réactionnel (2) et/ou l'étape c) d'injection d'au moins un gaz (7) au sein du milieu réactionnel, dans le bioréacteur anaérobie (1).

4. Procédé de méthanation selon l'une quelconque des revendications 1 à 3 **caractérisé en ce que** l'on injecte ledit au moins un gaz (7) au sein dudit milieu réactionnel (2) au cours de l'étape c) à une pression P1 supérieure d'au moins 10 mbar à la pression limite Pl de formation des bulles de gaz dans un liquide.

5. Procédé de méthanation selon l'une quelconque des revendications 1 à 4 **caractérisé en ce que** l'on injecte ledit au moins un gaz au sein dudit milieu réactionnel (2) au cours de l'étape c) à une pression P1 comprise entre PI+10 mbars et PI+500 mbars.

6. Procédé de méthanation selon l'une quelconque des revendications 1 à 5 **caractérisé en ce que** l'on injecte, au cours de l'étape c), un premier gaz (7) consistant en du dihydrogène H₂ et un deuxième gaz consistant en du biogaz, et **en ce que** l'on injecte au moins quatre moles de dihydrogène H₂ pour une mole de dioxyde de carbone CO₂ contenu dans le biogaz injecté.

7. Procédé de méthanation selon l'une quelconque des revendications 1 à 5 **caractérisé en ce que** l'on injecte, au cours de l'étape c), un premier gaz (7) consistant en du syngas.

8. Procédé de méthanation selon l'une quelconque des revendications 1 à 7 **caractérisé en ce que**, au cours de l'étape a), on alimente, en continu, ledit bioréacteur anaérobie (1) avec un substrat liquide (3) consistant en un effluent de méthaniseur.

9. Procédé de méthanation selon l'une quelconque des revendications 1 à 7 **caractérisé en ce que** ledit bioréacteur anaérobie (1) consiste en un méthaniseur et **en ce que**, au cours de l'étape a), on alimente, en continu, ledit méthaniseur avec un substrat liquide (3) consistant en un effluent industriel et/ou en un effluent agricole et/ou un effluent issu d'assainissement des eaux de station d'épuration.

10. Procédé de méthanation selon la revendication 9 **caractérisé en ce que** l'on injecte dans ledit méthaniseur, au cours de l'étape c), uniquement un premier gaz consistant en du dihydrogène H₂ ou en du syngas.

11. Installation (100) pour la mise en oeuvre du procédé de méthanation biologique et en continu de dihydrogène H₂ et d'un gaz choisi parmi le dioxyde de carbone CO₂ et le monoxyde de carbone CO, en vue de la production d'un biogaz riche en méthane CH₄, selon l'une quelconque des revendications 1 à 10, ladite installation (100) comportant, au moins, un bioréacteur anaérobie (1) comprenant un milieu réactionnel (2), contenant au moins un substrat liquide (3) avec des matières organiques à dégrader, et une flore microbienne comprenant au moins des microorganismes méthanogènes hydrogénotrophes, aptes à catalyser la transformation de H₂ et de CO₂ ou de H₂ et de CO en CH₄, ladite installation (100) comportant également au moins des moyens d'alimentation dudit bioréacteur anaérobie (1) en substrat liquide (3), des moyens d'extraction du biogaz (9) riche en méthane CH₄ produit, et un module de filtration (40) dudit milieu réactionnel (2) au travers d'une membrane de filtration (4) que comporte ledit module (40), ladite membrane de filtration (4) présentant un seuil de coupure compris entre 0,01 et 0,2 µm, apte à retenir une partie du milieu réactionnel (2) contenant la flore microbienne et des macromolécules présentant une taille supérieure audit seuil de coupure, et à laisser passer un digestat (5), ladite membrane de filtration (4) étant reliée à des moyens d'évacuation dudit digestat (5), ladite installation étant **caractérisée en ce qu'**elle comporte encore des premiers moyens d'injection, au sein dudit milieu réactionnel (2) que comporte ledit bioréacteur anaérobie (1), d'au moins un premier gaz consistant en du dihydrogène H₂ ou en du syngas, et, optionnellement, des deuxièmes moyens d'injection d'un deuxième gaz choisi parmi le monoxyde de carbone CO, le dioxyde de carbone CO₂ et le biogaz, le(s)dit(s) moyen(s) d'injection comportant, d'une part, un contacteur membranaire (80) comprenant une membrane microporeuse hydrophobe (8) et, d'autre part des moyens de contrôle et de maintien de la pression d'injection P1 dudit au moins un premier gaz au sein dudit milieu réactionnel (2) à une pression supérieure à la pression limite Pl de formation des bulles de gaz injecté dans un liquide.

## Patentansprüche

1. Verfahren zum kontinuierlichen und biologischen Methanisieren von Diwasserstoff H₂ und einem Gas, das aus Kohlendioxid CO₂ und Kohlenmonoxid CO ausgewählt ist, im Hinblick auf die Herstellung von Biomethan CH₄ oder einem an Methan CH₄ reichen Biogas in einem anaeroben Bioreaktor (1), umfassend ein Reaktionsmedium (2), das mindestens ein flüssiges Substrat (3) mit abzubauenden organischen Materialien enthält, und eine mikrobielle Flora, umfassend mindestens hydrotrophe methanogene Mikroorganismen, die geeignet ist, um die Umwandlung von H₂ und CO₂ oder von H₂ und CO in CH₄ zu katalysieren, wobei das Verfahren **dadurch gekennzeichnet ist, dass** es mindestens die folgenden Schritte umfasst:
a) kontinuierliches Versorgen des anaeroben Bioreaktor (1) mit dem flüssigen Substrat (3);
b) kontinuierliches Filtern des Reaktionsmediums (2) durch eine Filtrationsmembran (4), die eine Trenngrenze zwischen 0,01 µm und 0,2 µm vorweist, die geeignet ist, um einen Teil des Reaktionsmediums (2) zurückzuhalten, der die mikrobielle Flora und Makromoleküle enthält, die eine Größe vorweisen, die größer als die Trenngrenze ist, und Ablassen eines Gärguts (5);
c) kontinuierliches Injizieren, in das Reaktionsmedium (2), das von dem anaeroben Bioreaktor (1) aufgewiesen wird, mindestens eines ersten Gases (7), bestehend aus Diwasserstoff H₂ oder Synthesegas, letzteres umfassend mindestens Kohlenmonoxid CO und Diwasserstoff H₂, und optional eines zweiten Gases, das aus Kohlenmonoxid CO, Kohlendioxid CO₂ und Biogas ausgewählt ist, wobei das mindestens eine Gas (7) durch einen Membrankontaktor (80), umfassend eine hydrophobe mikroporöse Membran (8), in das Reaktionsmedium (2) injiziert wird, wobei die Injektion des mindestens einen Gases (7) innerhalb des Reaktionsmediums (2) bei einem Druck P1 über dem Grenzdruck Pl einer Blasenbildung des mindestens einen Gases (7) in einer Flüssigkeit durchgeführt wird;
d) Rückgewinnen von Biomethan CH₄ oder einem an Methan CH₄ reichen Biogas, das innerhalb des anaeroben Bioreaktors (1) hergestellt wird.

2. Methanisierungsverfahren nach Anspruch 1,
**dadurch gekennzeichnet, dass** Schritt b) des Filterns des Reaktionsmediums (2) und/oder Schritt c) des Injizierens mindestens eines Gases (7) innerhalb des Reaktionsmediums mittels einer Rezirkulationsschleife (6) außerhalb des anaeroben Bioreaktors (1) durchgeführt wird.

3. Methanisierungsverfahren nach Anspruch 1,
**dadurch gekennzeichnet, dass** Schritt b) des Filterns des Reaktionsmediums (2) und/oder Schritt c) des Injizierens mindestens eines Gases (7) innerhalb des Reaktionsmediums in dem anaeroben Bioreaktor (1) durchgeführt wird.

4. Methanisierungsverfahren nach einem der Ansprüche 1 bis 3,
**dadurch gekennzeichnet, dass** das mindestens eine Gas (7) während Schritt c) bei einem Druck P1, der mindestens 10 mbar größer als der Grenzdruck Pl für die Gasblasenbildung in einer Flüssigkeit ist, innerhalb des Reaktionsmediums (2) injiziert wird.

5. Methanisierungsverfahren nach einem der Ansprüche 1 bis 4,
**dadurch gekennzeichnet, dass** das mindestens eine Gas während Schritt c) bei einem Druck P1 zwischen PI+10 mbar und PI+500 mbar innerhalb des das Reaktionsmediums (2) injiziert wird.

6. Methanisierungsverfahren nach einem der Ansprüche 1 bis 5,
**dadurch gekennzeichnet, dass** während Schritt c) ein erstes Gas (7), bestehend aus Diwasserstoff H₂, und ein zweites Gas, bestehend aus Biogas, injiziert wird, und dass mindestens vier Mol H₂ für ein Mol Kohlendioxid CO₂, das in dem injizierten Biogas enthalten ist, injiziert werden.

7. Methanisierungsverfahren nach einem der Ansprüche 1 bis 5,
**dadurch gekennzeichnet, dass** während Schritt c) ein erstes Gas (7), bestehend aus Synthesegas, injiziert wird.

8. Methanisierungsverfahren nach einem der Ansprüche 1 bis 7,
**dadurch gekennzeichnet, dass** der anaerobe Bioreaktor (1) während Schritt a) mit einem flüssigen Substrat (3) bestehend aus einem Methanisierungsabwasser kontinuierlich versorgt wird.

9. Methanisierungsverfahren nach einem der Ansprüche 1 bis 7,
**dadurch gekennzeichnet, dass** der anaerobe Bioreaktor (1) aus einem Methanisierer besteht und dass der Methanisierer während Schritt a) mit einem flüssigen Substrat (3) kontinuierlich versorgt wird, bestehend aus einem Industrieabwasser und/oder einem landwirtschaftlichen Abwasser und/oder einem Abwasser aus einer Reinigung des Wassers einer Kläranlage.

10. Methanisierungsverfahren nach Anspruch 9,
**dadurch gekennzeichnet, dass** während Schritt c) nur ein erstes Gas, bestehend aus Diwasserstoff H₂ oder Synthesegas, in den Methanisierer injiziert wird.

11. Einrichtung (100) zum Durchführen des Verfahrens der kontinuierlichen und biologischen Methanisierung von Diwasserstoff H₂ und einem Gas, das aus Kohlendioxid CO₂ und Kohlenmonoxid CO ausgewählt ist, im Hinblick auf die Herstellung eines an Methan CH₄ reichen Biogases, nach einem der Ansprüche 1 bis 10, wobei die Einrichtung (100) mindestens einen anaeroben Bioreaktor (1) aufweist, umfassend ein Reaktionsmedium (2), das mindestens ein flüssiges Substrat (3) mit abzubauenden organischen Materialien enthält, und eine mikrobielle Flora, umfassend mindestens hydrotrophe methanogene Mikroorganismen, die geeignet sind, um die Umwandlung von H₂ und CO₂ oder H₂ und CO in CH₄ zu katalysieren, wobei die Einrichtung (100) auch mindestens Mittel zum Versorgen des anaeroben Bioreaktors (1) mit flüssigem Substrat (3), Mittel zum Extrahieren des an Methan CH₄ reichen hergestellten Biogases (9), und ein Filtermodul (40) des Reaktionsmediums (2) durch eine Filtermembran (4) aufweist, die von dem Modul (40) aufgewiesen wird, wobei die Filtermembran (4) eine Trenngrenze zwischen 0,01 und 0,2 µm vorweist, die geeignet ist, um einen Teil des Reaktionsmediums (2) zurückzuhalten, der die mikrobielle Flora und Makromoleküle enthält, die eine Größe über der Trenngrenze vorweisen, und um ein Gärgut (5) passieren zu lassen, wobei die Filtermembran (4) mit Mitteln zum Ablassen des Gärguts (5) verbunden ist, wobei die Einrichtung
**dadurch gekennzeichnet ist, dass** sie auch erste Mittel zum Injizieren innerhalb des Reaktionsmediums (2), das von dem anaeroben Bioreaktor (1) aufgewiesen wird, mindestens eines ersten Gases, das aus Diwasserstoff H₂ oder Synthesegas besteht, und optional zweite Mittel zum Injizieren eines zweiten Gases aufweist, das aus Kohlenmonoxid CO, Kohlendioxid CO₂ und Biogas ausgewählt ist, wobei das/die Injektionsmittel einerseits einen Membrankontaktor (80), umfassend eine hydrophobe mikroporöse Membran (8), und andererseits Mittel zum Steuern und Halten des Injektionsdrucks P1 des mindestens einen ersten Gases innerhalb des Reaktionsmediums (2) bei einem Druck über dem Grenzdruck Pl für die Blasenbildung von Gas umfasst, das in eine Flüssigkeit injiziert wird.

## Claims

1. A method for the continuous biological methanation of dihydrogen H₂ and a gas selected from carbon dioxide CO₂ and carbon monoxide CO, for the production of biomethane CH₄ or biogas rich in methane CH₄, in an anaerobic bioreactor (1) comprising a reaction medium (2), containing at least one liquid substrate (3) with organic matter to be degraded, and a microbial flora, comprising at least hydrogenotrophic methanogenic microorganisms, capable of catalyzing the conversion of H₂ and CO₂ or H₂ and CO into CH₄, said process being **characterized in that** it comprises, at least, the following steps:
a) Said anaerobic bioreactor (1) is continuously supplied with the liquid substrate (3);
b) Said reaction medium (2) is continuously filtered through a filtration membrane (4) with a cut-off threshold of between 0.01 µm and 0.2 µm, capable of retaining part of the reaction medium (2) containing the microbial flora and macromolecules having a size greater than said cut-off threshold, and a digestate (5) is discharged;
c) At least one first gas (7) consisting of dihydrogen H₂ or syngas, the latter comprising at least carbon monoxide CO and dihydrogen H₂, and, optionally, a second gas selected from carbon monoxide CO, carbon dioxide CO₂ and biogas, is continuously injected into said reaction medium (2) contained in said anaerobic bioreactor (1), said at least one gas (7) being injected into said reaction medium (2) through a membrane contactor (80) comprising a hydrophobic microporous membrane (8), the injection of said at least one gas (7) into said reaction medium (2) being carried out at a pressure P1 higher than the limit pressure Pl for bubble formation of said at least one gas (7) in a liquid;
d) Biomethane CH₄, or biogas rich in methane CH₄, produced within said anaerobic bioreactor (1) is recovered.

2. The method for methanation according to claim 1, **characterized in that** step b) of filtering the reaction medium (2) and/or step c) of injecting at least one gas (7) into the reaction medium is carried out by means of a recirculation loop (6) external to said anaerobic bioreactor (1).

3. The method for methanation according to claim 1, **characterized in that** step b) of filtering the reaction medium (2) and/or step c) of injecting at least one gas (7) into the reaction medium is carried out in the anaerobic bioreactor (1).

4. The method for methanation according to any one of claims 1 to 3, **characterized in that** said at least one gas (7) is injected into said reaction medium (2) during step c) at a pressure P1 at least 10 mbar higher than the limit pressure Pl for gas bubble formation in a liquid.

5. The method for methanation according to any one of claims 1 to 4, **characterized in that** said at least one gas is injected into said reaction medium (2) during step c) at a pressure P1 of between PI+10 mbar and PI+500 mbar.

6. The method for methanation according to any one of claims 1 to 5, **characterized in that,** during step c), a first gas (7) consisting of dihydrogen H₂ and a second gas consisting of biogas are injected, and **in that** at least four moles of dihydrogen H₂ are injected for one mole of carbon dioxide CO₂ contained in the injected biogas.

7. The method for methanation according to any one of claims 1 to 5, **characterized in that** a first gas (7) consisting of syngas is injected during step c).

8. The method for methanation according to any one of claims 1 to 7, **characterized in that,** during step a), said anaerobic bioreactor (1) is continuously supplied with a liquid substrate (3) consisting of a methanizer effluent.

9. The method for methanation according to any one of claims 1 to 7, **characterized in that** said anaerobic bioreactor (1) consists of a methanizer and **in that**, during step a), said methanizer is continuously supplied with a liquid substrate (3) consisting of an industrial effluent and/or an agricultural effluent and/or an effluent from wastewater treatment plants.

10. The method for methanation according to claim 9, **characterized in that** only a first gas consisting of dihydrogen H₂ or syngas is injected into said methanizer during step c).

11. A facility (100) for carrying out the method for continuous biological methanation of dihydrogen H₂ and a gas selected from carbon dioxide CO₂ and carbon monoxide CO, for producing a biogas rich in methane CH₄, according to any one of claims 1 to 10, said facility (100) comprising at least one anaerobic bioreactor (1) comprising a reaction medium (2), containing at least one liquid substrate (3) with organic matter to be degraded, and a microbial flora comprising at least hydrogenotrophic methanogenic microorganisms, capable of catalyzing the conversion of H₂ and CO₂ or H₂ and CO into CH₄, said facility (100) also comprising at least means for supplying said anaerobic bioreactor (1) with liquid substrate (3), means for extracting the biogas (9) rich in methane CH₄ produced, and a filtration module (40) for filtering said reaction medium (2) through a filtration membrane (4) contained in said module (40), said filtration membrane (4) having a cut-off threshold of between 0.01 and 0.2 µm, capable of retaining a portion of the reaction medium (2) containing the microbial flora and macromolecules having a size greater than said cut-off threshold, and allowing a digestate (5) to pass through, said filtration membrane (4) being connected to means for discharging said digestate (5), said facility being **characterized in that** it further comprises first means for injecting, within said reaction medium (2) of said anaerobic bioreactor (1), at least a first gas consisting of dihydrogen H₂ or syngas, and, optionally, second means for injecting a second gas selected from carbon monoxide CO, carbon dioxide CO₂ and biogas, said injection means comprising, on the one hand, a membrane contactor (80) comprising a hydrophobic microporous membrane (8) and, on the other hand, means for controlling and maintaining the injection pressure P1 of said at least one first gas within said reaction medium (2) at a pressure higher than the limit pressure Pl for the bubble formation of gas injected into a liquid.
